Europäisches Patentamt

European Patent Office

Office européen des brevets

(11) Veröffentlichungsnummer: **0 381 045**

**A2**

(12) # EUROPÄISCHE PATENTANMELDUNG

(21) Anmeldenummer: 90101460.5

(22) Anmeldetag: 25.01.90

(51) Int. Cl.⁵: **C07C 315/02, C07C 317/14**

(30) Priorität: 01.02.89 DE 3902894

(43) Veröffentlichungstag der Anmeldung:
08.08.90 Patentblatt 90/32

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Stumpp, Michael, Dr.**
**An der Marlach 13**
**D-6705 Deidesheim(DE)**
Erfinder: **Neumann, Peter, Dr.**
**Poststrasse 28**
**D-6800 Mannheim 31(DE)**

(54) **Verfahren zur Herstellung von Bis-(4-chlorphenyl)-sulfon.**

(57) Verfahren zur Herstellung von Bis-(4-chlorphenyl)-sulfon, indem man Chlorbenzol bei 150 bis 280° C und 1,3 bis 6 bar mit Chlorbenzolsulfonsäure in Gegenwart von Polyphosphorsäure oder einem Gemisch aus Phosphorpentoxid und einer nichtaromatischen Sulfonsäure umsetzt.

EP 0 381 045 A2

EP 0 381 045 A2

## Verfahren zur Herstellung von Bis-(4-chlorphenyl)-sulfon

Diese Erfindung betrifft ein neues und verbessertes Verfahren zur Herstellung von Bis-(4-chlorphenyl)-sulfon durch Umsetzung von Chlorbenzol mit Chlorbenzolsulfonsäure in Gegenwart von Polyphosphorsäure oder einem Gemisch aus Phosphorpentoxid und einer nichtaromatischen Sulfonsäure bei erhöhten Temperaturen und Drücken.

Bis-(4-chlorphenyl)-sulfon ist ein wichtiges Zwischenprodukt, das hauptsächlich für die Herstellung aromatischer Polysulfone und zur Synthese von Bis-(4-aminophenyl)-sulfon, das man sowohl für die Lepratherapie als auch zur Härtung von Epoxidharzen benötigt, verwendet wird. Voraussetzung für diese Einsatzgebiete ist eine hohe Reinheit des Bis-(4-chlorphenyl)-sulfons.

Aus DE-A-27 04 972 ist die Herstellung von Bis-(4-chlorphenyl)-sulfon durch Friedel-Crafts-Reaktion von 4-Chlor-benzolsulfochlorid mit Chlorbenzol, z.B. mit Eisen-III-chlorid als Katalysator und in Chlorbenzol als Lösungsmittel bei ca. 140°C, bekannt. Aus J. Am. Chem. Soc. 76, 5491-5494 (1954) ist bekannt, daß Eisen-III-chlorid bei dieser Reaktionstemperatur auch als Chlorierungsmittel auf Chlorbenzol wirkt, so daß das auch als Lösungsmittel eingesetzte und wiedergewonnene Chlorbenzol erhebliche Anteile an Dichlorbenzolen enthält, die eine aufwendige Aufarbeitung notwendig machen, da auch Dichlorbenzole mit Chlorbenzolsulfochlorid zum Sulfon reagieren können.

Aus der DE-A-22 52 571 ist bekannt, daß man aus Chlorbenzol und Chlorbenzol-sulfonsäure bei Temperaturen von 220 bis 260°C und überatmosphärischem Druck Bis-(4-chlorphenyl)-sulfon in guten Ausbeuten, allerdings bei verhältnismäßig langen Reaktionszeiten erhält.

Laut J. Org. Chem. 32, 2931-33 (1967) wird bei 80°C auch unter Verwendung von Polyphosphorsäure keine Umsetzung von Chlorbenzol mit p-Toluolsulfonsäure beobachtet.

Aus Synthesis 4, 323-325 (1984) ist die Umsetzung von Chlorbenzol mit p-Toluolsulfonsäure in Anwesenheit eines Gemisches aus Phosphorpentoxid und Methansulfonsäure bei 80°C bekannt. Unter diesen Bedingungen wird lediglich ein Umsatz von 9 % erzielt.

Der Erfindung lag daher die Aufgabe zugrunde, ein neues und verbessertes Verfahren zur Herstellung von Bis-(4-chlorphenyl)-sulfon aus Chlorbenzol und Chlorbenzolsulfonsäure zu entwickeln, das den vorgenannten Nachteilen abhilft.

Demgemäß wurde ein neues und verbessertes Verfahren zur Herstellung von Bis-(4-chlorphenyl)-sulfon gefunden, welches dadurch gekennzeichnet ist, daß man Chlorbenzol bei 150 bis 280°C und 1,3 bis 6 bar mit Chlorbenzolsulfonsäure in Anwesenheit von Polyphosphorsäure oder eines Gemisches aus Phosphorpentoxid und einer nichtaromatischen Sulfonsäure umsetzt.

Das erfindungsgemäße Verfahren kann wie folgt durchgeführt werden:

Man kann die Reaktion in Anwesenheit reiner Polyphosphorsäure, vorteilhafterweise jedoch in einem Gemisch aus Phosphorpentoxid und einer nichtaromatischen Sulfonsäure, wie Methansulfonsäure, Fluorsulfonsäure oder Trifluormethansulfonsäure durchführen, da dieses Gemisch besser zu handhaben ist als die hochviskose Polyphosphorsäure. Besonders bevorzugt ist die Verwendung von einem Gemisch aus Phosphorpentoxid und Methansulfonsäure.

Zur Umsetzung wird vorteilhafterweise Chlorbenzolsulfonsäure zusammen mit Chlorbenzol und Polyphosphorsäure oder einem Gemisch aus Phosphorpentoxid mit Methansulfonsäure oder einer anderen nichtaromatischen Sulfonsäure in einem Autoklaven plaziert. Das druckdichte Reaktionssystem wird auf 150 bis 280°C, vorzugsweise auf 200 bis 250°C hochgeheizt, wobei sich der Innendruck auf 1,3 bis 6 bar, vorzugsweise 3 bis 5 bar erhöht. Anschließend wird ca. 5 bis 20 Stunden unter diesen Bedingungen gerührt und die Reaktionslösung nach Abkühlen auf Eiswasser gekippt. Vor der Filtration des Rohproduktes wird gegebenenfalls überschüssiges Chlorbenzol durch Phasentrennung oder durch azeotrope Destillation entfernt.

Das Chlorbenzol wird in der Regel in stöchiometrischer Menge bezüglich der p-Chlorbenzolsulfonsäure oder in einem Überschuß von 0,001 bis 50 Mol% verwendet. Das Gewicht der eingesetzten Polyphosphorsäure beträgt das 2- bis 50-fache, in der Regel das 20-fache des Gewichts der eingesetzten Chlorbenzolsulfonsäure. Bei der Verwendung von einem Gemisch aus Phosphorpentoxid und nichtaromatischer Sulfonsäure wird ein Molverhältnis von 0,001:1 bis 10:1, vorzugsweise 0,01:1 bis 5:1, besonders bevorzugt 0,05:1 bis 0,5:1 verwendet.

Durch Verwendung von Polyphosphorsäure oder einem Gemisch aus Phosphorpentoxid und Methansulfonsäure konnte die Reaktionstemperatur, im Vergleich zur Umsetzung ohne Zusatz eines dieser Kondensationshilfsmittel, erheblich gesenkt werden.

Ferner wurde gefunden, daß dieser Zusatz und die damit verbundene Temperaturabsenkung auch einen vorteilhaften Einfluß auf die Isomerenverteilung der gebildeten Dichlordiphenylsulfone ausübt. Folgende

2

Isomerenverteilungen wurden gefunden:

| | 4,4'-Isomer | 2,4'-Isomer | 3,4'-Isomer |
|---|---|---|---|
| ohne Zusatz eines Kondensationsmittels | 79 % | 7,5 % | 13,5 % |
| mit Zusatz von Polyphosphorsäure | 85 % | 7,7 % | 7,3 % |
| mit Zusatz von Phosphorpentoxid/Methansulfonsäure | 91 % | 4,0 % | 5,0 % |

Das nach Filtration erhaltene Rohprodukt kann nach bekannten Verfahren, beispielsweise durch selektives Waschen mit einem Lösungsmittel, wie z.B. Aceton, durch Umkristallisation aus einem Alkohol, wie Methanol, Ethanol, n-Propanol oder n-Butanol oder aus Chlorbenzol durch fraktionierte Kristallisation oder durch Zentrifugieren gereinigt werden.

Beispiele

Beispiel 1

In einem 5 l-Reaktionskessel aus Hastelloy C4 wurden 500 g (2,6 Mol) p-Chlorbenzolsulfonsäure, 281 g (2,6 Mol) Chlorbenzol und 2000 g Polyphosphorsäure plaziert und unter Rühren auf 200 °C aufgeheizt, wobei sich der Innendruck auf 3 bar erhöhte. Nach 10 Stunden wurde auf Raumtemperatur abgekühlt und das Gemisch mit 10 l Eiswasser versetzt und das Rohprodukt nach Erwärmen auf 80 °C abfiltriert und zwei Mal mit 1 l Wasser gewaschen und getrocknet.
Ausbeute: 462 g (74 % d. Th.)
Isomerenverteilung:
4,4': 85 %
2,4' : 7,7 %
3,4': 7,3 %

Beispiel 2

Man arbeitete analog Beispiel 1, unter Verwendung von 1800 g Methansulfonsäure und 200 g Phosphorpentoxid anstelle von 2000 g Polyphosphorsäure.
Ausbeute: 438 g (70% d. Th.)
Isomerenverteilung:
4,4': 91 %
2,4': 4,0 %
3,4': 5,0 %

Beispiel 3 (Vergleichsbeispiel)

Man arbeitete analog Beispiel 1, jedoch ohne Zusatz eines Kondensationhilfsmittels bei 240 °C und Entfernung des Reaktionswassers durch azeotrope Destillation mit Chlorbenzol unter Rückführung von Chlorbenzol.
Ausbeute: 425 g (68% d. Th.)
Isomerenverteilung:
4,4': 79 %
2,4': 7,5 %
3,4': 13,5%

Beispiel 4 (Vergleichsbeispiel)

Man arbeitete analog Beispiel 1, jedoch ohne Zusatz eines Kondensationhilfsmittels bei 200 °C.

## EP 0 381 045 A2

Ausbeute: 218 g (35 % d. Th.)
Isomerenverteilung:
4,4': 84 %
2,4': 8,0 %
3,4': 8,0 %


**Ansprüche**

1. Verfahren zur Herstellung von Bis-(4-chlorphenyl)-sulfon, dadurch gekennzeichnet, daß man Chlorbenzol bei 150 bis 280°C und 1,3 bis 6 bar mit Chlorbenzolsulfonsäure in Gegenwart von Polyphosphorsäure oder einem Gemisch aus Phosphorpentoxid und einer nichtaromatischen Sulfonsäure umsetzt.

2. Verfahren nach Anspruch 1, dadurch gekennzeichnet, daß man als nichtaromatische Sulfonsäure Methansulfonsäure, Fluorsulfonsäure oder Trifluormethansulfonsäure verwendet.

3. Verfahren nach Anspruch 1, dadurch gekennzeichnet, daß man die Umsetzung bei 200 bis 250°C durchführt.

4. Verfahren nach Anspruch 1, dadurch gekennzeichnet, daß man die Umsetzung bei 3 bis 5 bar durchführt.